(19) **Europäisches Patentamt**

**European Patent Office**

**Office européen des brevets**

(11) **EP 0 749 961 B1**

(12) **FASCICULE DE BREVET EUROPEEN**

(45) Date de publication et mention de la délivrance du brevet:
**13.06.2001 Bulletin 2001/24**

(51) Int Cl.[7]: **C07C 319/08**, C07C 321/04, B01J 21/04, B01J 27/232

(21) Numéro de dépôt: **96401326.2**

(22) Date de dépôt: **19.06.1996**

(54) **Procédé de préparation de méthylmercaptan**

Verfahren zur Herstellung von Methylmercaptan

Process for the preparation of methylmercaptan

(84) Etats contractants désignés:
**AT BE CH DE DK ES FI FR GB GR IE IT LI LU NL PT SE**

(30) Priorité: **23.06.1995 FR 9507571**

(43) Date de publication de la demande:
**27.12.1996 Bulletin 1996/52**

(73) Titulaire: **AVENTIS ANIMAL NUTRITION S.A.**
**92160 Antony (FR)**

(72) Inventeurs:
• **Ponceblanc, Hervé**
**75018 Paris (FR)**
• **Tamburro, François**
**69001 Lyon (FR)**

(74) Mandataire: **Mérigeault, Shona et al**
**Aventis CropScience**
**Département Propriété Industrielle**
**BP 9163**
**69263 Lyon Cedex 09 (FR)**

(56) Documents cités:
**FR-A- 1 161 066**        **FR-A- 2 477 538**

• **REACTION KINETICS AND CATALYSIS LETTERS, vol. 38, no. 1, 1989, BUDAPEST, HU, pages 199-203, XP002014519 V.M. KUDENKOV, ET AL.: "Synthesis of methylmercaptan in the presence of base catalysts"**

Remarques:
Le dossier contient des informations techniques présentées postérieurement au dépôt de la demande et ne figurant pas dans le présent fascicule.

## Description

La présente invention concerne un procédé de préparation du méthylmercaptan amélioré. Elle concerne particulièrement un procédé de préparation du méthylmercaptan à partir de méthanol et d'hydrogène sulfuré. Elle concerne également les catalyseurs de préparation présentant une stabilité améliorée.

Il est connu depuis très longtemps de préparer le méthylmercaptan à partir de méthanol et d'hydrogène sulfuré. On trouve une description de ce type de réaction par exemple selon Sabatier et Mailhe en 1910 dans un compte rendu de l'académie des sciences 150 823-6, 1569-72 et 1217-21.

La réaction a été décrite en présence de divers catalyseurs surtout à base d'alumine éventuellement dopée avec divers métaux parmi lesquels on peut citer les alcalins, les alcalinoterreux, le sulfure de cadmium, le sulfure d'antimoine, l'oxyde d'étain, l'acide phosphotungstique, phosphomolybdique ainsi que le tungstate de potassium.

La réaction est décrite par passage dans un seul réacteur ou dans plusieurs réacteurs successifs. Ainsi selon le brevet GB 1 417 532 il est connu de préparer le méthylmercaptan par réaction du méthanol et de l'hydrogène sulfuré selon un rapport molaire de l'hydrogène sulfuré au méthanol compris entre 1.10 et 2.5 dans une série d'au moins trois réacteurs catalytiques. Le catalyseur employé est de préférence une alumine dopée avec du tungstate de potassium, la température de réaction est maintenue entre 320 et 370°C.

Les produits secondaires de réaction dans ce type de procédé sont constitués essentiellement par le diméthylsulfure et le dioxyde de carbone. La formation de diméthylsulfure augmente avec la température de réaction, mais le degré de transformation du méthanol augmente aussi avec la température, ainsi un équilibre doit pouvoir être trouvé entre la formation de diméthylsulfure et la conversion du méthanol de départ.

Il est encore connu selon les articles des auteurs russes Kudenkov, Paukshtis et Mashkina paru dans *react. Kinet. Catal*. Lett., *Vol 38*, *No 1, 199-203 (1989)* d'utiliser comme catalyseurs de réaction des alumines dopées soit au tungstate de potassium, soit au carbonate de potassium, soit à la potasse soit enfin à la soude à des températures comprises entre 360 et 450°C. La meilleure sélectivité en méthylmercaptan est obtenue avec le tungstate de potassium que ce soit à 360 ou à 400°C. Le meilleur taux de conversion du méthanol est obtenu lorsque la température est la plus haute c'est à dire à 500°C. Si l'on calcule le rendement en méthylmercaptan en fonction de la température et du type de catalyseur on est amené à utiliser une températre comprise entre 400 et 450°C sans distinction dans la nature du catalyseur à utiliser.

Selon un autre article des mêmes auteurs paru dans *Kinetika i Kataliz*, *Vol. 29*, *No. 5*, *pp. 1174-1180, September-October 1988* comparant l'activité et la sélectivité des catalyseurs à base d'alumine dopée au tungstate de potassium ou au carbonate de potassium, il est apparu qu'à une température de 360°C la sélectivité en méthylmercaptan était équivalente avec un catalyseur dopé au tungstate ou au carbonate.

Il est apparu selon la présente invention de façon tout à fait étonnante que l'activité et la sélectivité de ces deux types de catalyseurs étaient tout à fait différentes lorsqu'ils étaient utilisés à des températures bien inférieures à celles utilisées dans l'art antérieur.

La présente invention concerne donc un procédé de préparation de méthylmercaptan à partir de méthanol et d'hydrogène sulfuré en phase vapeur en présence d'un système catalytique caractérisé en ce que le catalyseur utilisé est un carbonate alcalin déposé sur alumine et qu'il est utilisé à une température inférieure à 350°C.

Le carbonate alcalin utilisé comme catalyseur de réaction est choisi parmi les carbonates de sodium, de potassium ou de cesium. On préfère utiliser le carbonate de potassium. La quantité pondérale de carbonate alcalin déposée sur alumine varie en fonction de la nature du métal alcalin, elle sera néanmoins de préférence comprise entre 2 et 20% en poids. En ce qui concerne le carbonate de potassium cette quantité sera de préférence comprise entre 2 et 10%. Selon une meilleure manière de mettre en oeuvre l'invention on utilisera une quantité pondérale de carbonate de potassium d'environ 6,4% déposé sur alumine.

La température de réaction est encore plus préférentiellement comprise entre 230 et 330°C et tout particulièrement comprise entre 280 et 310°C. On entend par température de réaction la température mesurée à l'entrée du réacteur. La température d'entrée est en général inférieure à la température de sortie, car la réaction est exothemique.

La pression réactionnelle est de préférence comprise entre 8 et 15 bars (8 à 15 $10^5$ pascals).

Le catalyseur est de préférence réparti dans un système catalytique contenant au moins trois réacteurs.

Dans le premier réacteur encore appelé convertisseur on introduit de préférence la totalité de l'hydrogène sulfuré, le méthanol étant introduit de façon séquentielle au niveau de chacun des autres réacteurs. Le rapport molaire global entre l'hydrogène sulfuré introduit et le méthanol introduit est compris entre 1,5 et 2,5. Le premier catalyseur utilisé dans le convertisseur est de préférence de l'alumine. Son rôle consiste essentiellement à transformer le diméthylsulfure recyclé et provenant du dernier réacteur, en présence d'hydrogène sulfuré neuf, en méthylmercaptan.

A la sortie du convertisseur les gaz issus sont introduits dans le premier réacteur et en même temps est alimenté le méthanol, celui ci est introduit en partie sous forme liquide et en partie sous forme gazeuse de façon à stabiliser la température du réacteur. En effet la vaporisation du méthanol permet de consommer une partie des calories apportées par la réaction qui est exothermique. La charge de méthanol entrant, méthanol provenant du réacteur précédent et nouveau méthanol introduit, est notamment de plus en plus importante dans chacun des réacteurs au fur et à mesure que la réaction progresse. Pour chacun des réacteurs le rapport molaire entre l'hydrogène sulfuré et l'apport partiel de méthanol baisse au fur et à mesure de la progression de la réaction et est notamment compris entre 15 et 3.

Le rapport global de l'hydrogène sulfuré au méthanol est toujours compris dans les limites préalablement indiquées. Ces valeurs permettent d'obtenir une selectivité élevée en méthylmercaptan au détriment de la formation de diméthylsulfure.

Après le dernier réacteur le mélange réactionnel est introduit dans le finisseur contenant le même catalyseur que la série des réacteurs précédents mais d'un volume égal ou supérieur. En sortie du finisseur les gaz de réaction constitués de méthylmercaptan, de diméthylsulfure, de méthanol et d'hydrogène sulfuré et de gaz divers sont séparés selon le brevet GB 1 417 532 précité.

L'invention sera plus complètement décrite à l'aide des exemples suivants qui ne doivent pas être considérés comme limitatifs de l'invention.

EXEMPLE 1

Préparation du catalyseur de formule $K_2CO_3/Al_2O_3$ à 2% en poids équivalent à un nombre de cation alcalin de 28,9 mmole/100g de catalyseur.

Une solution de 120ml est préparée en dissolvant 4,08g de $K_2CO_3$ (exempt de sodium) dans de l'eau permutée. Le pH de la solution finale est de 11,66. 200g d'alumine Procalyse SPHERALITE 505 sont imprégnées à sec par la solution précédente. Le catalyseur ainsi préparé est activé à 470°C. Le catalyseur est constitué de 2% en poids de $K_2CO_3$.

EXEMPLE 2

Préparation du catalyseur de formule $K_2CO_3/Al_2O_3$ à 6,4% en poids équivalent à un nombre de cation alcalin de 92,6 mmole/100g de catalyseur.

Le catalyseur de l'exemple 2 est préparé selon la méthode décrite à l'exemple 1, en utilisant 13,68g de $K_2CO_3$ pour imprégner 200g d'alumine. Le pH de la solution avant imprégnation est de 11,6.

EXEMPLE 3

Préparation du catalyseur de formule $K_2CO_3/Al_2O_3$ à 10% en poids équivalent à un nombre de cation alcalin de 144,7 mmole/100g de catalyseur.

Le catalyseur de l'exemple 3 est préparé selon la méthode décrite à l'exemple 1, en utilisant 22,22g de $K_2CO_3$ pour imprégner 200g d'alumine. Le pH de la solution avant imprégnation est de 11,75.

EXEMPLE 4

Préparation du catalyseur de formule $Na_2CO_3/Al_2O_3$ à 4,9% en poids équivalent à un nombre de cation alcalin de 92,4 mmole/100g de catalyseur.

Le catalyseur de l'exemple 3 est préparé selon la méthode décrite à l'exemple 1, en utilisant 10g de $Na_2CO_3$ pour imprégner 200g d'alumine. Le pH de la solution avant imprégnation est de 10,79.

EXEMPLE 5

Préparation du catalyseur de formule $Cs_2CO_3/Al_2O_3$ à 15,1% en poids équivalent à un nombre de cation alcalin de 92,5 mmole/100g de catalyseur.

Le catalyseur de l'exemple 3 est préparé selon la méthode décrite à l'exemple 1, en utilisant 35,49g de $Cs_2CO_3$ pour imprégner 200g d'alumine. Le pH de la solution avant imprégnation est de 11,02.

EXEMPLE COMPARATIF 1

Ce catalyseur est constitué d'alumine seule.

Le catalyseur de l'exemple comparatif 1 est constitué d'alumine SPHERALITE 505, non imprégnée et recuite selon la même procédure qu'à l'exemple 1.

EXEMPLE COMPARATIF 2

Préparation du catalyseur de formule $K_2WO_4/Al_2O_3$ à 14% en poids équivalent à un nombre de cation alcalin de 92,5 mmole/100g de catalyseur.

Le catalyseur de l'exemple comparatif 2 est préparé selon la méthode décrite à l'exemple 1, en utilisant 40,23g de $K_2WO_4$, $2H_2O$ pour imprégner 200g d'alumine. Le pH de la solution avant imprégnation est de 11.

EXEMPLE COMPARATIF 3

Préparation du catalyseur de formule $Na_2WO_4/Al_2O_3$ à 13,6% en poids équivalent à un nombre de cation alcalin de 92,5 mmole/100g de catalyseur.

Le catalyseur de l'exemple comparatif 3 est préparé selon la méthode décrite à l'exemple 1, en utilisant 36g de $Na_2WO_4$, $2H_2O$ pour imprégner 200g d'alumine. Le pH de la solution avant imprégnation est de 9,88.

EXEMPLE 6

Test catalytique des catalyseurs des exemples 1 à 5 et comparatifs 1 à 3.

Les performances catalytiques des catalyseurs décrits dans les exemples 1 à 5 et comparatifs 1 à 3, ont été déterminées dans les conditions suivantes.

Un lit de 90ml, constitué de catalyseur sous forme de billes présentant un diamètre d'environ 3mm non dilué, est contenu dans un réacteur de 35cm de haut et de 27,3mm de diamètre intérieur.

Les gaz d'entrée du réacteur sont constitués d'un mélange de méthanol, d'hydrogène sulfuré, de méthylmercaptan, de diméthylsulfure et d'eau, dans les proportions molaires suivantes: 6,5/70,5/11,5/4/7,5%. Ils traversent d'abord un lit de 100ml de billes corindon de façon à être préchauffés à la température de la réaction. La pression est maintenue dans le réacteur à 10 bars. La température de réaction est maintenue à 320°C.

La VVH ou vitesse volumique horaire, calculée dans les conditions normales de température et de pression est de $6667h^{-1}$ soit un temps de contact de 0,54s.

La vitesse volumique horaire, VVH est définie comme le rapport :

$$\frac{\text{Débit total des réactifs dans les conditions normales}}{\text{volume de catalyseur}}$$

Le taux de conversion du méthanol, Xg est défini comme le rapport:

$$\frac{\text{Nombre de moles de méthanol converti}}{\text{Nombre de moles de méthanol à l'entrée}} \times 100$$

La sélectivité en produit i, Si est définie comme le rapport:

$$\frac{\text{Nombre de moles de produit i formé}}{\text{Nombre de moles de méthanol converti}} \times 100$$

Le rendement en produit i, Ri est défini comme le prosuit Xg X Si.

Les performances catalytiques des exemples 1 à 5 et comparatifs 1 à 3, mesurées après 100 heures sous réactifs sont reportées dans le tableau 1 suivant:

TABLEAU 1

| Exemples | Catalyseur (% en poids) | Xg (%) | SMSH (%) | SDMS (%) | $SCO_2$ (%) | RMSH (%) | RDMS (%) |
|---|---|---|---|---|---|---|---|
| C1 | alumine seule | 100 | 60 | 40 | 0 | 60 | 40 |
| 1 | 2% $K_2CO_3$ | 94,0 | 82,5 | 17,5 | 0 | 77,6 | 16,5 |
| 2 | 6,4% $K_2CO_3$ | 93,0 | 93,0 | 5,0 | 2,0 | 86,5 | 4,7 |
| 3 | 10% $K_2CO_3$ | 78,5 | 89,5 | 9,5 | 1,0 | 70,3 | 7,5 |
| 4 | 4,9% $Na_2CO_3$ | 87,0 | 89,0 | 10,0 | 1,0 | 77,4 | 8,7 |

TABLEAU 1   (suite)

| Exemples | Catalyseur (% en poids) | Xg (%) | SMSH (%) | SDMS (%) | $SCO_2$ (%) | RMSH (%) | RDMS (%) |
|---|---|---|---|---|---|---|---|
| 5 | 15,1% $Cs_2CO_3$ | 92,0 | 94,0 | 5,0 | 1,0 | 86,5 | 11,0 |
| C2 | 14% $K_2WO_4$ | 75,0 | 94,0 | 4,0 | 2,0 | 70,5 | 3,0 |
| C3 | 13,6%$Na_2WO_4$ | 86,0 | 87,0 | 5,0 | 8,0 | 74,8 | 4,3 |

Les conversions de méthanol et rendements en MSH, correspondants aux exemples 1 à 4 et comparatifs 1 et 2, mesurés à différents temps de marche, pour une température de réaction de 370°C (conditions de vieillissements accéléré) sont reportés dans le tableau 2 suivant :

TABLEAU 2

| Exemples | catalyseur (% en poids) | durée en heure | Xg (%) | RMSH (%) | -Xg/$\Delta$t (%) pour 100 h |
|---|---|---|---|---|---|
| C1 | alumine seule | 100 | 100 | 70,0 | 0 |
| | | 150 | 100 | 63,0 | |
| | | 250 | 100 | 62,0 | |
| | | 400 | 100 | 57,0 | |
| 1 | 2%$K_2CO_3$ | 100 | 100 | 79,0 | 1 |
| | | 150 | 100 | 77,5 | |
| | | 250 | 98,0 | 77,0 | |
| | | 400 | 97,0 | 75,0 | |
| 2 | 6,4%$K_2CO_3$ | 100 | 98,0 | 86,0 | 0,6 |
| | | 250 | 98,0 | 86,5 | |
| | | 400 | 97,5 | 87,0 | |
| | | 500 | 96,0 | 87,6 | |
| | | 600 | 95,0 | 87,5 | |
| 3 | 10%$K_2CO_3$ | 100 | 95,0 | 77,0 | 1,6 |
| | | 250 | 93,0 | 77,5 | |
| | | 400 | 91,0 | 78,0 | |
| | | 500 | 88,0 | 77,0 | |
| | | 600 | 87,0 | 75,0 | |
| 4 | 4,9%$Na_2CO_3$ | 250 | 100 | 88,0 | 1,4 |
| | | 400 | 97,5 | 86,0 | |
| | | 500 | 97,0 | 87,0 | |
| | | 600 | 95,0 | 86,0 | |
| C2 | 14%$K_2WO_4$ | 250 | 90,0 | 85,0 | 5 |
| | | 400 | 85,0 | 82,0 | |
| | | 500 | 77,0 | 78,5 | |
| | | 550 | 75,0 | 77,0 | |

<u>EXEMPLES 6 A 10 ET COMPARATIFS 4 A 8</u>

Un essai industriel est réalisé sur une batterie de 7 réacteurs précédée par un convertisseur. Le convertisseur est chargé avec de l'alumine SPHERALITE 505® . Les 7 réacteurs sont chargés soit avec le catalyseur de l'exemple 2 contenant 6,4% $K_2CO_3$, soit avec le catalyseur de l'exemple comparatif 2. On introduit dans le convertisseur 5000 kg/h d'un mélange contenant en poids:

84 % d'hydrogène sulfuré
6% de méthanol
5 % de diméthylsulfure

5 % de méthylmercaptan

On introduit dans chacun des 7 réacteurs 300 kg/h de méthanol. On réalise plusieurs essais à différentes températures d'entrée des gaz dans le réacteur. Les résultats sont indiqués dans le tableau 3 suivant:

TABLEAU 3

| Exemples | Température (°C) | Catalyseur | Xg | SMSH (%) | SDMS (%) | RMSH (%) |
|---|---|---|---|---|---|---|
| 6 | 230 | 6,4% $K_2CO_3$ | 63,68 | 99,40 | 0,60 | 63,30 |
| C4 | 230 | 14% $K_2WO_4$ | 42,97 | 99,85 | 0,15 | 42,90 |
| 7 | 280 | 6,4% $K_2CO_3$ | 82,77 | 94,44 | 5,56 | 78,17 |
| C5 | 280 | 14% $K_2WO_4$ | 63,27 | 98,56 | 1,44 | 62,36 |
| 8 | 310 | 6,4% $K_2CO_3$ | 91,93 | 85,54 | 14,46 | 78,64 |
| C6 | 310 | 14% $K_2WO_4$ | 72,89 | 96,13 | 3,87 | 70,07 |
| 9 | 340 | 6,4% $K_2CO_3$ | 99,15 | 66,27 | 33,73 | 65,70 |
| C7 | 340 | 14% $K_2WO_4$ | 82,29 | 90,48 | 9,52 | 74,46 |
| 10 | 370 | 6,4% $K_2CO_3$ | 100,00 | 37,77 | 62,23 | 37,77 |
| C8 | 370 | 14% $K_2WO_4$ | 92,07 | 78,53 | 21,47 | 72,30 |

Il est évident à la lecture des tableaux 1 à 3 que si l'on veut tenir compte de la stabilité du catalyseur au cours du temps et de son efficacité sur le rendement en méthylmercaptan il est très avantageux d'utiliser un catalyseur à base de $K_2CO_3$ et à une température comprise entre 230 et 330°C et de préférence entre 280°C et 310°C.

**Revendications**

1. Procédé de préparation de méthylmercaptan à partir de méthanol et d'hydrogène sulfuré en phase vapeur en présence d'un système catalytique caractérisé en ce que le catalyseur utilisé est un carbonate alcalin déposé sur alumine et qu'il est utilisé à une température inférieure à 350°C.

2. Procédé selon la revendication 1 caractérisé en ce que le carbonate alcalin utilisé est choisi parmi les carbonates de sodium, de potassium ou de césium.

3. Procédé selon la revendication 2 caractérisé en ce que le carbonate alcalin est le carbonate de potassium.

4. Procédé selon la revendication 1 caractérisé en ce que la quantité pondérale de carbonate alcalin déposée sur alumine est comprise entre 2 et 20% en poids.

5. Procédé selon les revendications 4 et 3 caractérisé en ce que la quantité pondérale de carbonate de potassium déposée sur alumine est comprise entre 2 et 10% et est de préférence d'environ 6,4%.

6. Procédé selon la revendication 1 caractérisé en ce que la température de réaction est comprise entre 230 et 330°C.

7. Procédé selon la revendication 6 caractérisé en ce que la température de réaction est comprise entre 280 et 310°C.

8. Procédé selon la revendication 1 caractérisé en ce que le rapport molaire de l'hydrogène sulfuré au méthanol est compris entre 1,5 et 2,5.

9. Procédé selon la revendication 1 caractérisé en ce que la pression réactionnelle est comprise entre 8 et 15 bars (8 à 15 $10^5$ Pa).

10. Procédé selon la revendication 1 caractérisé en ce que le catalyseur est reparti dans un système comprenant au moins trois réacteurs.

**Claims**

1. Process for preparing methyl mercaptan from methanol and hydrogen sulphide in the vapour phase in the presence of a catalytic system, characterized in that the catalyst used is an alkali metal carbonate deposited on alumina and in that it is used at a temperature below 350°C.

2. Process according to claim 1, characterized in that the alkali metal carbonate used is chosen from sodium carbonate, potassium carbonate and caesium carbonate.

3. Process according to claim 2, characterized in that the alkali metal carbonate is potassium carbonate.

4. Process according to claim 1, characterized in that the weight amount of alkali metal carbonate deposited on alumina is between 2% and 20% by weight.

5. Process according to claims 3 and 4, characterized in that the weight amount of potassium carbonate deposited on alumina is between 2% and 10% and is preferably about 6.4%.

6. Process according to claim 1, characterized in that the reaction temperature is between 230°C and 330°C.

7. Process according to claim 6, characterized in that the reaction temperature is between 280°C and 310°C.

8. Process according to claim 1, characterized in that the molar ratio of hydrogen sulphide to methanol is between 1.5 and 2.5.

9. Process according to claim 1, characterized in that the reaction pressure is between 8 bar and 15 bar (8 to 15 $\times$ $10^5$ Pa).

10. Process according to claim 1, characterized in that the catalyst is distributed in a system comprising at least three reactors.

**Patentansprüche**

1. Verfahren zur Herstellung von Methylmercaptan ausgehend von Methanol und Schwefelwasserstoff in der Dampfphase in Gegenwart eines katalytischen Systems, dadurch gekennzeichnet, daß der verwendete Katalysator ein auf Aluminiumoxid abgeschiedenes Alkalicarbonat ist und daß er bei einer Temperatur unter 350°C eingesetzt wird.

2. Verfahren nach Anspruch 1, dadurch gekennzeichnet, daß das verwendete Alkalicarbonat unter den Carbonaten von Natrium, Kalium oder Cäsium ausgewählt wird.

3. Verfahren nach Anspruch 2, dadurch gekennzeichnet, daß das Alkalicarbonat Kaliumcarbonat ist.

4. Verfahren nach Anspruch 1, dadurch gekennzeichnet, daß die auf Aluminiumoxid abgeschiedene gewichtsmäßige Menge von Alkalicarbonat zwischen 2 und 20 Gew.-% beträgt.

5. Verfahren nach den Ansprüchen 4 und 3, dadurch gekennzeichnet, daß die auf Aluminiumoxid abgeschiedene gewichtsmäßige Menge von Kaliumcarbonat zwischen 2 und 10% beträgt und vorzugsweise ungefähr 6,4% ist.

6. Verfahren nach Anspruch 1, dadurch gekennzeichnet, daß die Reaktionstemperatur zwischen 230 und 330°C liegt.

7. Verfahren nach Anspruch 6, dadurch gekennzeichnet, daß die Reaktionstemperatur zwischen 280 und 310°C liegt.

8. Verfahren nach Anspruch 1, dadurch gekennzeichnet, daß das Molverhältnis von Schwefelwasserstoff zu Methanol zwischen 1,5 und 2,5 liegt.

9. Verfahren nach Anspruch 1, dadurch gekennzeichnet, daß der Reaktionsdruck zwischen 8 und 15 bar (8 bis 15 $\cdot 10^5$ Pa) beträgt.

**10.** Verfahren nach Anspruch 1, dadurch gekennzeichnet, daß der Katalysator in einem System, das mindestens drei Reaktoren umfaßt, verteilt ist.